# EUROPEAN PATENT APPLICATION

(11) **EP 4 732 890 A1**
(43) Date of publication of application: **29.04.2026**
(21) Application number: 25204088.6
(22) Date of filing: 23.09.2025
(51) Int. Cl.: A61N 1/05, A61N 1/36

(54) **A SYSTEM FOR CONTROLLING SELECTIVE NERVE STIMULATION OF NEURAL TISSUE OF A SUBJECT**

(30) Priority: 03.10.2024 US 202418905283
(71) Applicant: Stichting IMEC Nederland, 5656 AE Eindhoven (NL); The Feinstein Institutes for Medical Research, Manhasset, NY 11030 (US)
(72) Inventor: SCHNEPEL, Philipp, 5654 GG Eindhoven (NL); MIHAJLOVIC, Vojkan, 5643 AZ Eindhoven (NL); ROSSETTI, Nicolo, 5654 AN Eindhoven (NL); SONG, Weiguo, Manhasset, 11030 (US); ZANOS, Stavros, Manhasset, 11030 (US)
(74) Representative: AWA Sweden AB

(57) **Abstract**

A system for controlling selective stimulation of neural tissue of a subject is provided. Said system comprises a first electrode arrangement, comprising a plurality of electrodes, configured to be arranged in in relation to a first longitudinal position of the neural tissue, a second electrode arrangement, comprising at least one electrode and configured to be arranged at a second longitudinal position of the neural tissue, and a stimulation signal generating unit. The stimulation signal generating unit is configured to generate a first intermittent current waveform comprising a first pulse and a second intermittent current waveform comprising a second pulse, for causing interferential stimulation of a plurality of nerve fibers of the neural tissue. The stimulation signal generating unit is further configured to generate and output a blocking pulse to the second electrode arrangement, for temporally selectively blocking propagation of signals through the neural tissue at the second longitudinal position.

## Description

### Technical field

The present description relates to controlling selective nerve stimulation of neural tissue of a subject. In particular, the present description relates to a system and a method for controlling selective nerve stimulation of neural tissue of a subject.

### Background

Neurostimulation may be used for controlling a function of an organ or part of a body of a living being, such as a human being, e.g., for alleviating or treating a number of conditions of the human being. Neurostimulation may involve a control of electrical signals transmitted through a nerve, so as to trigger or block signals transmitted by the nerve.

It is known that peripheral neural fiber properties such as diameter and myelination have an impact on activation thresholds, with larger myelinated fibers being easier to excite as compared to smaller, unmyelinated fibers. This poses challenges when trying to stimulate smaller fibers before large fibers, due to the different activation thresholds.

Stimulation of a target may be provided using a plurality of electrodes arranged in different locations in relation to the target. In particular, if it is desired to target a specific part of the nerve, such as a fascicle or nerve fiber(s) within the fascicle, use of the plurality of electrodes is advantageous. Typically, a signal provided to a nerve will attenuate with a depth into the nerve such that a maximum electric field is provided at an outer part of the nerve, in epineurium. In particular, since the epineurium has low conductive properties, large signals are required for reaching deep into the nerve. However, signals from two pairs of electrodes, or from a plurality of pairs of electrodes, may be provided so as to interfere within the nerve and selectively provide a large electric field within the nerve, forming interferential stimulation of the nerve. This implies that a part of the nerve inside the epineurium may be stimulated while using modest signal levels.

However, there is still a need to improve interferential stimulation to allow accurate stimulation of a target such that control of neural signals is provided to desired portion(s) of neural tissue. Indeed, there is a need for better stimulation control to avoid unwanted or harmful neural and physiological responses.

### Summary

An objective of the present description is to provide accurate control of spatially and temporally selective stimulation of neural tissue.

This and other objectives are at least partly met by the invention as defined in the independent claims. Preferred embodiments are set out in the dependent claims.

According to a first aspect, there is provided a system for controlling selective stimulation of neural tissue of a subject, said system comprising, a first electrode arrangement, comprising a plurality of electrodes, wherein different electrodes of the plurality of electrodes are configured to be arranged in different electrode locations in relation to a first longitudinal position of the neural tissue. The system further comprises a second electrode arrangement, comprising at least one electrode and configured to be arranged at a second longitudinal position of the neural tissue, and a stimulation signal generating unit, wherein the stimulation signal generating unit is configured to generate a first intermittent current waveform comprising a first pulse and a second intermittent current waveform comprising a second pulse, wherein the first pulse comprises a first frequency and the second pulse comprises a second frequency, wherein the second frequency is different from the first frequency, wherein the stimulation signal generating unit is configured to output the first intermittent current waveform to a first pair of electrodes of the first electrode arrangement and the second intermittent current waveform to a second pair of electrodes of the first electrode arrangement, for causing interferential stimulation of a plurality of nerve fibers of the neural tissue, wherein the interferential stimulation of each of the nerve fibers of the plurality of nerve fibers is configured to activate stimulation of the nerve fiber at a specific time point, for initiating propagation of signals through the neural tissue towards the second longitudinal position, wherein the stimulation signal generating unit is configured to generate a blocking pulse and to output the blocking pulse to the at least one electrode of the second electrode arrangement, for temporally selectively blocking propagation of signals through the neural tissue at the second longitudinal position.

The system may be used for controlling stimulation of neural tissue of a subject wherein the subject may be a living being. **It** should be realized that the system may be used for controlling stimulation of neural tissue of a human being but that the system may likewise be used for controlling stimulation of neural tissue of an animal.

Neural tissue is found in the brain, spinal cord and nerves. As used herein, the term "nerve" should be construed as a bundle of nerve fibers enclosed by a sheath called the epineurium. The nerve may form part of the peripheral nervous system and may therefore also be called a peripheral nerve. The nerve is configured to transmit electrical impulses within the body along axons, which may be bundled in fascicles in the nerve. The electrical impulses may be provided as action potentials transmitted along the axons.

**It** should be realized that, as used herein the term "neural tissue" may be used interchangeably with the terms "nerve", "nervous tissue", "nervous system", "spinal cord" and "brain".

The neural tissue, such as a nerve, may have a longitudinal extension forming an elongate structure. The neural tissue may be configured to propagate signals along the longitudinal extension. **It** should be realized that the first longitudinal position and the second longitudinal position refer to separate positions along the longitudinal extension of the neural tissue.

The plurality of electrodes of the first electrode arrangement are configured to be arranged in relation to the first longitudinal position. This implies that the electrodes of the first electrode arrangement may be configured to output electrical signals into the neural tissue at the first longitudinal position for interacting with the neural tissue at the first longitudinal position.

Similarly, the at least one electrode of the second electrode arrangement is configured to be arranged in relation to the second longitudinal position. This implies that the electrodes of the second electrode arrangement may be configured to output electrical signals into the neural tissue at the second longitudinal position for interacting with the neural tissue at the first longitudinal position.

It should further be realized that the electrodes being arranged in relation to the first longitudinal position and the second longitudinal position, respectively, need not necessarily be arranged in relation to a single cross-section of the neural tissue.

According to an embodiment, the electrodes within the first pair of electrodes of the first electrode arrangement may be slightly displaced in relation to each other along the longitudinal extension of the neural tissue, and the electrodes within the second pair of electrodes of the first electrode arrangement may be slightly displaced in relation to each other along the longitudinal extension of the neural tissue. Thus, the first longitudinal position should not be understood as a single cross-section of the neural tissue but may rather correspond to a (small) extension along the longitudinal extension of the neural tissue.

The electrodes of the first and second pairs may be arranged at corresponding cross-sections of the neural tissue, such that a first electrode of the first pair and a first electrode of the second pair are arranged in relation to a same first cross-section of the neural tissue, whereas a second electrode of the first pair and a second electrode of the second pair are arranged in relation to a same second cross-section of the neural tissue.

The first intermittent current waveform and the second intermittent current waveform may thus each define electric fields along a common longitudinal direction of the neural tissue extending into the neural tissue so as to form interferential stimulation.

The at least one electrode of the second electrode arrangement is configured to be arranged at a second longitudinal position which is separate from the first longitudinal position. Thus, even though the first longitudinal position may have an extension along the longitudinal extension of the neural tissue, the second longitudinal position does not overlap with the first longitudinal position.

According to another embodiment, the first pair of electrodes and the second pair of electrodes are arranged at different locations of a circumference of a nerve. Thus, the first intermittent current waveform and the second intermittent current waveform may mainly define electric fields based on the waveforms in different portions of a cross-section of the nerve. The first intermittent current waveform and the second intermittent current waveform may interfere within the nerve to stimulate the nerve through interferential stimulation.

Both electrodes of the first pair of electrodes may be associated with a first part of the circumference of the nerve and both electrodes of the second pair of electrodes may be associated with a second part of the circumference of the nerve, wherein the second part is different from and non-overlapping with the first part. This implies that the portions of the cross-section of the nerve in which the first intermittent current waveform and the second intermittent current waveform, respectively, define electric fields are quite different so as to allow interferential stimulation deep within the nerve without necessarily providing stimulation in undesired locations of the nerve.

Alternatively, the electrodes of the first pair of electrodes and the electrodes of the second pair of electrodes may be alternatingly arranged around the circumference of the nerve, such that an electrode of the first pair of electrodes is arranged between the electrodes of the second pair of electrodes along the circumference of the nerve. This implies that the first intermittent current waveform and the second intermittent current waveform may define electric fields through the nerve with a large area of overlap of the electric fields based on the first intermittent current waveform and the second intermittent current waveform, respectively.

All of the electrodes of the first pair of electrodes and the second pair of electrodes may be provided in relation to a common cross-section of the nerve.

The plurality of electrodes of the first electrode arrangement may comprise one or more pre-defined pairs of electrodes to be used for providing the first and second pulse, respectively. However, according to an alternative embodiment, the electrodes forming a pair of electrodes used for providing the first and second pulse, respectively, may be dynamically selected among the plurality of electrodes.

Furthermore, according to an alternative embodiment, the first electrode arrangement may comprise three or more pairs of electrodes to which the signal generator can output intermittent current waveforms, such that the multiple intermitted current waveforms form interferential stimulation within the neural tissue.

In other words, the stimulation signal generating unit may thus be configured to generate a intermittent current waveform comprising a pulse for each of the pairs of electrodes, and output the intermittent current waveform to each of the pairs of electrodes, respectively, for causing interferential stimulation of a plurality of nerve fibers of the neural tissue.

The system is configured to provide a first intermittent current waveform and a second intermittent current waveform. Thus, a signal may be intermittently provided to the first pair of electrodes and a signal may be intermittently provided to the second pair of electrodes. The first waveform and the second waveform may each comprise a plurality of pulses. The first waveform and the second waveform need not be continuous over the plurality of pulses with the first waveform and/or the second waveform being modulated to form pulses of interferential stimulation with sufficient amplitude to activate the nerve. Rather, the first pair of electrodes and the second pair of electrodes may only receive signals at time instances when stimulation of the neural tissue is to take place.

Thus, the first intermittent current waveform may provide a non-zero signal or high amplitude signal within the first pulse and may provide a zero-level signal between pulses within a sequence of first pulses. Also, the second intermittent current waveform may provide a non-zero signal or high amplitude signal within the second pulse and may provide a zero-level signal between pulses within a sequence of second pulses. **It** should be realized that instead of a zero-level signal, a low amplitude signal may be provided between the first pulses and between the second pulses, respectively. The low amplitude signal may have such a low amplitude that no stimulation of the neural tissue takes place through interferential stimulation and the amplitude may also be low so as not to draw a lot of energy.

The first intermittent current waveform and the second intermittent current waveform may thus be called intermittent in that the waveforms intermittently have sufficiently high amplitude to cause interferential stimulation and therebetween have a very low or zero amplitude to reduce power consumption. **It** should further be realized that the waveforms may comprise single pulses, respectively. Thus, each time a pulse is to be output, the stimulation signal generating unit may be controlled to generate a waveform comprising a single pulse and the control of generation of the single pulse is not related to generation of other pulses.

Thus, thanks to the stimulation generating unit being configured to provide intermittent current waveforms, the stimulation of the neural tissue may be provided in an energy-efficient manner. The stimulation generating unit needs only output signals during the first pulses and the second pulses, respectively.

This implies that the system facilitates use of an implanted device (for which energy saving is particularly important in order to ensure long lifetime of the implanted device or avoid frequent charging of the implanted device) with stimulation paradigms that include neural tissue being stimulated in very long sessions (such as up to 30 minutes) comprising sequences of pulses being provided in plural periods separated by "off time" when no stimulation is provided.

The stimulation generating unit is configured to generate current waveforms for providing a current to the neural tissue. The current can be converted to a voltage within the neural tissue based on transconductance of tissue such that an action potential for stimulating the neural tissue is formed.

Thanks to the system being configured to generate the first waveform comprising the first frequency to the first pair of electrodes of the first electrode arrangement and the second waveform comprising the second frequency to the second pair of electrodes of the first electrode arrangement, the system is able to define an interference between the first waveform and the second waveform in the neural tissue. When the first waveform and the second waveform interfere, the interference may form a varying signal having a frequency corresponding to the difference in frequency between the first frequency and the second frequency. This frequency of the interference between the first and second waveforms may be referred to as a beat frequency.

The first frequency and the second frequency may be selected to be high frequencies, whereas the beat frequency represents a low frequency lower than the first frequency and the second frequency. Thus, the first waveform and the second waveform may include a first frequency and a second frequency, respectively. Further, an amplitude of the first waveform and an amplitude of the second waveform may be smaller than an amplitude of an interferential signal with the beat frequency, such that a sufficiently large signal (amplitude) for stimulating the nerve is only provided by the interferential signal.

The blocking pulse is configured to temporally and selectively block propagation of signals through the neural tissue. This is done by temporally inhibiting the neural tissue, which results in preventing propagation through the neural tissue at the second longitudinal position. The stimulation signal generating unit is configured to generate a blocking pulse and to output the blocking pulse to the at least one electrode of the second electrode arrangement.

Thanks to the blocking pulse temporally and selectively inhibiting propagation of signals through the neural tissue, a temporally selective control of the neural stimulation may be achieved. In other words, the blocking pulse may temporally inhibit propagation of selected signals, and thereby prevent unwanted or harmful neural or physiological responses as side effects to a desired stimulation.

The difference in time of activation for each nerve fiber, in other words, that the interferential stimulation of each of the nerve fibers of the plurality of nerve fibers is configured to activate the nerve fiber at a specific time point, may be due to a difference in when an interferential stimulation signal based on the interference of the first and second intermittent current waveforms activates the nerve fiber. The interferential stimulation signal may form an activation function in the neural tissue, the activation function being the second derivative of electrical potential generated due to current injection by the first and second intermittent current waveforms. The activation function may activate a particular nerve fiber when the activation function reaches an activation threshold. The difference in time of activation for each nerve fiber may be due to a difference in time when the activation function reaches the activation threshold.

Interferential stimulation leads to amplitude modulation which may be larger towards a center of the nerve than at edges of the nerve which are closer to the electrodes. Hence, fibers closer to the central regions of the nerve may be activated later in time than fibers arranged closer to the electrodes. Additionally, the amplitude modulation may be focused on an edge, or another part of the nerve. Hence, fibers closer to the amplitude-modulated region of the nerve may be activated later in time than fibers arranged further away from it. In certain examples a majority of the nerve fibers in the nerve will be activated (including nerve fibers close to, adjacent or at the surface of the nerve) but at different time points. This implies that undesired propagation of signals in certain nerve fibers may be blocked while desired propagation of signals in other nerve fibers may be allowed due to activation time differences, such that undesired signals and desired signals do not reach the second longitudinal position at the same time point.

It should also be realized that if the system is configured to provide stimulation of neural tissue in a brain, the system may not be configured to differentiate between centrally arranged nerve fibers and nerve fibers arranged around the centrally arranged nerve fibers. Nevertheless, the system may still be configured to selectively control propagation of signals such that desired propagation may be allowed while undesired propagation may be blocked.

The interferential signal may advantageously provide different delays of the time point of fiber activation for different nerve fibers due to the time needed for the activation potential to reach a maximum amplitude. For instance, fiber type (A, B and C) and fiber properties such as diameter and myelination may have an impact on the activation thresholds. Larger myelinated fibers are easier to excite, and thus have a lower activation threshold as compared to smaller, unmyelinated fibers, which have a higher activation threshold. The interferential signal may cause fibers with a lower activation threshold to be activated prior to fibers with a higher activation threshold.

The interferential signal may be configured to provide an amplitude modulated signal, wherein an envelope curve of the signal may define the beat frequency. The interferential signal may include a sequence of cycles of increasing and decreasing amplitude within a single period of the envelope curve. Thus, a peak amplitude of respective cycles may be increasing in the sequence of cycles of the envelope curve. The interferential signal may be considered an amplitude modulated signal, with an amplitude that increases (or decreases) with time. Hence, the time delay of fiber activation may be enhanced by interferential intermittent current stimulation due to the amplitude modulation of the interferential signal, which can result in fiber activation at different cycles within the envelope curve.

Moreover, the fiber activation delay is different for fibers located in different regions of the neural tissue. In other words, the fibers located close to the origin (location of electrodes) of the interferential signal may have a shorter activation delay than fibers located further away from the origin of the interferential signal.

Additionally, a conduction velocity of an action potential, i.e., a speed at which the electrical signal propagates along the longitudinal extension of a nerve fiber, varies depending on the type of fiber and neuron. The speed of an action potential may be influenced by the diameter of the nerve fiber. Larger diameter axons may conduct signals faster than smaller diameter axons, due to less resistance to the flow of electrical current. The degree of myelination may also affect the speed of an action potential, as the myelin insulates the axon of the nerve fiber and allows for a more rapid transmission of the electrical signal.

It should be realized that the above mentioned factors affecting the conduction velocity are provided purely as examples and there may be other factors affecting the conduction velocity, such as channel composition, tissue thickness, temperature and more.

Thus, the diameter of the fiber and the degree of myelination around the axon of the fiber may affect both the activation threshold as well as the conduction speed of signals. In other words, larger myelinated fibers have a lower activation threshold and conduct signals faster compared to smaller myelinated fibers.

It should be realized that time delay between transmitted signals in different fibers is affected by both the time point of activation, as well as the conduction velocity of the action potential. For example, a larger fiber and a smaller fiber, located substantially in the same place within the nerve will have different time points of activation, due to the properties of the fiber and the amplitude modulated signal. The difference in conduction velocity will further increase the time delay between the smaller and larger fiber when the action potentials travel along a nerve.

In another example, a larger fiber is located closer to a center of the nerve, and a smaller fiber is located further away from the center. The smaller fiber may, due to its location, have an earlier time of activation than the larger fiber. However, due the difference in conduction velocity between the larger fiber and the smaller fiber, the time delay may decrease as the signals propagate along the nerve and the action potential of a larger nerve may precede the action potential of a smaller nerve at a certain distance.

Due to the multiple variables mentioned above affecting time activation and conduction velocity, the time delay may be either increased or decreased.

It is a realization of the first aspect that the above-described time delays of fiber activation for different fibers provides time windows wherein blocking of propagation of signals in nerve fibers may be provided without interfering with desired signals propagating towards a target. In other words, by timing the blocking pulse with the time window provided by the time delays of fiber activation between different fibers, it may be possible to block a single electrical signal propagating in a nerve fiber. Similarly, it may be possible to block all non-target signals, and only allowing for desired signals to continue propagating along respective nerve fibers, such as allowing only a single electrical signal propagating in a nerve fiber to continue propagating. Hence, the difference in activation timings across the neural tissue in combination with differences of propagation speeds of distinct fiber types may advantageously be leveraged to achieve functional selectivity, i.e., activation of only specific fiber types localized in a specific region of neural tissue or in specific fascicles leading to a specific functional response (neural or physiological).

The system according to the first aspect may utilize a combination of time-specific activation of nerve fibers due to the amplitude modulated interferential signal, and time-specific blocking of propagation. This combination provides temporally selective control of stimulation and signal propagation.

Furthermore, thanks to the use of the first and second pair of electrodes for causing interferential stimulation, the electrodes may be arranged externally to the neural tissue while providing stimulation in a desired location within the neural tissue.

The first and second electrode arrangements may be adapted to provide the respective electrodes close to or in contact with the neural tissue to be stimulated. This implies that stimulation signals may be output by the electrode arrangement directly into the neural tissue providing an accurate control of the stimulation within the nerve.

The neural tissue, such as a nerve, may comprise a plurality of fascicles. Within the nerve, a large number of fascicles may be provided, and each fascicle may include a large number of axons, such that the nerve may transmit signals to/from various parts of the body. For instance, the vagus nerve is a cranial nerve providing signals between the brain and parts of the body. The vagus nerve may be relatively easy to access and a system for stimulating a nerve that is to be implanted in the human being may therefore be advantageously arranged in relation to the vagus nerve for providing stimulation of the vagus nerve. However, since the vagus nerve provides signals to many different parts of the body, it is also important that stimulation of the nerve is controlled through interferential stimulation and the blocking pulse to selectively stimulate a portion of the nerve for providing a desired effect of the stimulation.

Stimulation of the neural tissue may correspond to providing a sufficiently large action potential to trigger a signal to be transmitted by the neural tissue or block a signal from being transmitted by the neural tissue. However, as used herein, the first and second pulse are used to trigger a signal to be transmitted by the neural tissue, while the blocking pulse is used to block a signal from being transmitted by the neural tissue.

In other words, the first and second pulses may be used to trigger a signal to be transmitted by the neural tissue to a target, while the blocking pulse may be used to block triggered signals from being transmitted by the neural tissue to a non-target.

Reference is made herein to "first" and "second", such as "first electrode arrangement" and "second electrode arrangement". It should be realized that items referred to as "first" are not the same item as items referred to as "second", unless stated otherwise. Thus, the first electrode arrangement and the second electrode arrangement are different from each other. The first pair of electrodes and the second pair of electrodes are different from each other. The first longitudinal position of the neural tissue and the second longitudinal position of the neural tissue are different from each other. The first intermittent current waveform and the second intermittent current waveform are different from each other. The first pulse and the second pulse are different from each other. The first frequency and the second frequency are different from each other.

The stimulation generating unit may comprise a plurality of stimulation generating modules with separate current generators, each dedicated to generating the first waveform and the second waveform respectively. This implies that the first waveform to be output to the first pair of electrodes and the second waveform to be output to the second pair of electrodes may be separately controlled to ensure that desired waveforms are output and that a waveform is not affected by generation of other waveforms in the stimulation generating unit. Hence, the interferential stimulation may be accurately controlled by separately controlling the first waveform and the second waveform.

The system advantageously provides spatially and temporally selective stimulation of neural tissue. Hence, accurate stimulation control and functional selectivity may be achieved. This may advantageously facilitate a reduction of side effects associated with off-target fiber stimulation.

Moreover, thanks to the system combining the advantages of the stimulation generating unit being configured to provide intermittent current waveforms and the temporally selective blocking, the stimulation of the neural tissue may be provided in an energy-efficient manner and preventing unwanted or harmful neural or physiological responses. Hence, improved stimulation control is provided.

According to an embodiment, the blocking pulse may be configured to hyperpolarize the neural tissue at the second longitudinal position, such that the propagation of signals through the neural tissue may be prevented.

Hyperpolarization refers to a change in a cell's membrane potential to further lower the membrane potential. Hyperpolarization may prevent (or makes it more difficult for) any stimulus supplied along an axon from triggering an action potential. Hyperpolarization prevents the conduction of an action potential as the membrane potential becomes so negative that an already travelling potential cannot cause a regeneration of the action potential in that point.

However, hyperpolarization may also be induced by applying a voltage, and thus changing a cell's membrane potential lowering (i.e., hyperpolarizing) the membrane potential. By sufficiently lowering the membrane potential, action potentials transmitted will not be further propagated as regeneration of the action potential fails, due to the negative membrane potential.

Thanks to the stimulation signal generating unit being configured to generate a blocking pulse configured to hyperpolarize the neural tissue at the second longitudinal position and to output the blocking pulse configured to hyperpolarize the neural tissue to the at least one electrode of the second electrode arrangement, temporally selectively blocking of propagation of signals through the neural tissue at the second longitudinal position may be achieved.

According to an embodiment, the stimulation signal generating unit is configured to generate the blocking pulse comprising at least a pulse width for defining a time duration during which temporally selectively blocking propagation of signals through the neural tissue occurs.

The blocking pulse may comprise a pulse shape wherein the pulse shape may be for example biphasic, linear, triangular or trapezoid. However, it should be realized that other pulse shapes may achieve substantially the same or similar effect as the provided examples of pulse shapes.

A timing of the blocking pulse and the time duration of the blocking pulse may be controlled to ensure that undesired propagation of signals, such as signals propagating in off-target fibers, may be blocked.

According to an embodiment, the blocking pulse is achieved by anodal blocking.

In one example, anodal blocking may be achieved by utilizing one electrode of a pair of electrodes of the first electrode arrangement together with the electrode of the second electrode arrangement. The blocking pulse may be output to an electrode in the second electrode arrangement acting as an anode and to an electrode in the first electrode arrangement acting as a cathode, for forming the blocking pulse at the second electrode arrangement. In other words, an electrode of the first electrode arrangement may act both as receiver of the first or the second intermittent current waveform and as receiver of the blocking pulse.

According to an embodiment, the second electrode arrangement comprises a plurality of electrodes, and wherein different electrodes of the plurality of electrodes are configured to be arranged in different electrode locations in relation to the second longitudinal position of the neural tissue.

The plurality of electrodes of the second electrode arrangement may comprise one or more pre-defined pairs of electrodes. However, according to an alternative embodiment, the electrodes forming a pair of electrodes may be dynamically selected among the plurality of electrodes.

In an example, an electrode or a group of electrodes may be selected from the plurality of electrodes of the second electrode arrangement. In other words, the second electrode arrangement may comprise a plurality of electrodes, and the stimulation signal generating unit may be configured to generate a blocking pulse and to output the blocking pulse to at least one electrode of the plurality of electrodes of the second electrode arrangement. Not all electrodes of the plurality of electrodes need thus be used at all times. For example, one electrode may be selected from the plurality of electrodes of the second electrode arrangement, and thus the stimulation signal generating unit may be configured to output the blocking pulse to the one selected electrode. In another example, a plurality, N, electrodes are selected and the stimulation signal generating unit may be configured to output the blocking pulse to the plurality of selected electrodes.

In the example wherein one electrode is selected, a return electrode, configured to close the current circuit, may be positioned away from the nerve to achieve monopolar blocking. In the example wherein a plurality of electrodes are selected the selected electrodes may be configured to be positive or negative, i.e., act as a source or as a return electrode in any possible combination.

The stimulation signal generating unit may be configured to output the blocking pulse to the pair of electrodes of the second electrode arrangement or to multiple pairs of electrodes of the second electrode arrangement.

Advantageously, outputting the blocking pulse to multiple pairs of electrodes of the second electrode arrangement may increase the area of blocking. In other words, an effective electrode size may be increased by selecting more electrodes. If all electrodes are selected, this corresponds to the single electrode case described above.

All electrodes of the plurality of electrodes of the second electrode arrangement may be controlled independently, i.e., providing different waveforms. All electrodes of the plurality of electrodes of the second electrode arrangement may be controlled in synchronization with each other, i.e., providing the same waveform.

According to an embodiment, the electrodes within the pair of electrodes of the second electrode arrangement may be slightly displaced in relation to each other along the longitudinal extension of the neural tissue. Thus, the second longitudinal position should not be understood as a single cross-section of the neural tissue but may rather correspond to a (small) extension along the longitudinal extension of the neural tissue.

The blocking pulse may thus define an electric field along a longitudinal direction of the neural tissue extending into the neural tissue so as to provide the blocking of propagation of signals at the second longitudinal position.

According to another embodiment, the electrodes of the second electrode arrangement are arranged at different locations of a circumference of a nerve. Thus, the blocking pulse may mainly define an electric field within a cross-section of the nerve.

All of the electrodes of the second electrode arrangement may be provided in relation to a common cross-section of the nerve.

According to an embodiment, the stimulation signal generating unit is configured to generate a repolarization pulse and to output the repolarization pulse to the at least one electrode of the second electrode arrangement following the blocking pulse.

This may be beneficial to ensure charge balancing for the at least one electrode of the second electrode arrangement.

An unbalanced charge may introduce an offset voltage build-up onto the electrode over time, in particular as the interface between the electrode and tissue is often very capacitive. The unbalanced charge can cause safety issues to the electrode and/or the tissue. For instance, the offset voltage build-up may form such a large electric field that electrical breakdown of water occurs. The offset voltage build-up may also cause the electrode to experience irreversible redox reactions such that a subject to the stimulation may be exposed to toxic substances.

According to an embodiment, the system may further comprise a third electrode arrangement, comprising at least one electrode and configured to be arranged at a third longitudinal position of the neural tissue.

This implies that the system may be configured to provide temporal control of propagation of signals in neural tissue in further positions. The third electrode arrangement provides an additional position in the neural tissue at which control of propagation of signals is provided. Thus, blocking of propagation of signals may be provided both at the second longitudinal position and the third longitudinal position providing a possibility for more accurate selection of signals that are allowed to propagate in the neural tissue.

The stimulation signal generating unit may be configured to generate a second blocking pulse and to output the second blocking pulse to the at least one electrode of the third electrode arrangement, for temporally selectively blocking propagation of signals through the neural tissue at the third longitudinal position. The second blocking pulse may be output simultaneously with the blocking pulse output to the at least one electrode of the second electrode arrangement or with partial or no overlap between the blocking pulses.

According to an embodiment, the second longitudinal position of the neural tissue is located in a first direction along the neural tissue in relation to the first longitudinal position and the third longitudinal position of the neural tissue is located in a second direction along the neural tissue in relation to the first longitudinal position, wherein the second direction is opposite to the first direction.

This implies that blocking of undesired propagation of signals in both directions along the neural tissue in relation to the first longitudinal position may be provided.

It should be realized that the system may comprise even further electrode arrangement arranged in further longitudinal positions of the neural tissue. Thus, one or more electrode arrangements for providing blocking pulses may be arranged in the first direction along the neural tissue and/or one or more electrode arrangements for providing blocking pulses may be arranged in the second direction along the neural tissue.

In a further example, the second and third electrode arrangements are configured to temporally selectively block propagation of signals through the neural tissue at a second and third longitudinal position, wherein the second and third longitudinal positions are located at different branches of a nerve. Thus, the stimulation of nerve fibers at the first longitudinal position may cause propagation of signals towards a location at which a nerve branches off into two separate branches. The second and third electrode arrangements may then be configured to control propagation of signals in each of the branches.

According to an embodiment, the stimulation signal generating unit comprises a plurality of current generators, wherein each current generator of the plurality of current generators may be configured to generate an intermittent current waveform or a blocking pulse, respectively.

According to an embodiment, the stimulation signal generating unit comprises a first current generator, configured to generate the first intermittent current waveform and a second current generator, configured to generate the second intermittent current waveform.

According to an embodiment, the stimulation signal generating unit comprises a third current generator, configured to generate the blocking pulse.

The system may thus comprise separate current generators for generating each of the first and second waveforms and the blocking pulse, respectively. This implies that generation of the first waveform to be output to the first pair of electrodes and generation of the second waveform to be output to the second pair of electrodes may be separately controlled. In addition, generation of the blocking pulse to be output to the second electrode arrangement may be separately controlled from the generation of the first and second waveforms. Hence, the stimulation of the neural tissue may be accurately controlled by separately controlling the first waveform, the second waveform, and the blocking pulse.

According to an embodiment, the system is configured to be at least partially implanted in the subject.

This facilitates use of the system for providing stimulation of the neural tissue to the living being in a convenient way. In particular, when the system is implanted within the body of a human being, the stimulation of the neural tissue may be provided with minimal effect of everyday life of the human being for providing the stimulation.

However, the implanted system may still be configured to communicate with an external device through wireless communication. The system may receive control signals from the external device for changing parameters of the stimulation, such as parameters defining the stimulation paradigm, such as to control a location within the neural tissue to be stimulated or a frequency, an amplitude, or a duration of a stimulation to be provided. The external device may be provided in a housing that may be worn by the living being or may be adapted for being arranged in vicinity of the living being. The implanted system may be configured to communicate with a communication unit, which may be provided in a housing worn by the living being or arranged in vicinity of the living being, wherein the communication unit may act as an intermediate device for allowing the implanted system to communicate with an external unit which may be remotely arranged. Thus, communication between the communication unit and the external unit may be provided via a telecommunication or computer network.

According to an embodiment, the stimulation generating unit is configured to generate a first frequency and a second frequency, respectively, being in a range of 100 Hz-1 MHz, such as 10 kHz-100 kHz.

Thus, the first stimulation generating unit and the second stimulation generating unit may generate signals having a relatively high frequency. The frequency of the signal in the first pulse and the frequency of the signal in the second pulse may preferably not be too high so that a relatively small difference in the frequencies may still be accurately defined. Thus, the frequency of the signal in the first pulse and the frequency of the signal in the second pulse may be lower than 1 MHz, such as lower than 100 kHz.

According to an embodiment, the stimulation signal generating unit is configured to generate the first intermittent current waveform and the second intermittent current waveform with a difference in frequency in a range of 1 Hz - 10 kHz, such as 1 - 5 kHz.

Thus, the beat frequency may be in the range of 1 Hz - 10 kHz or in the range of 1 - 5 kHz. The use of a beat frequency in these ranges may be suitable for providing stimulation of the neural tissue. If the beat frequency is very low, it may be difficult to accurately define the interferential signal in relation to high frequencies of the signal in the first pulse and the signal in the second pulse. Thus, the beat frequency may preferably be higher than 1 Hz or higher than 1 kHz. The beat frequency may be selected in relation to a desired stimulation of the neural tissue, such as in relation to a particular stimulation paradigm to be used.

According to an embodiment, the stimulation signal generating unit is configured to generate the first intermittent current waveform and the second intermittent current waveform with a time duration of the first pulse and the second pulse corresponding to a portion of a period of a beat frequency.

A portion of a period may for example correspond to half a period.

It should be appreciated that using half a period of the beat frequency is advantageous in that it is energy efficient while also enabling activation of all required fibers and avoiding double fiber activation.

According to an embodiment, the first pulse and the second pulse are sinusoidal signals.

This may be suitable waveforms of the first and second pulses for forming the interferential signal providing efficient stimulation of nerve fibers.

However, it should be realized that the waveforms need not necessarily be pure sinusoidal waveforms but may alternatively be, for instance, a pseudo-sinusoidal waveform, a modified sinusoidal waveform, or a multi-level waveform approximating a sinusoidal waveform. In addition, the waveforms may alternatively be periodic waveforms with another shape, such as a square wave or a trapezoidal wave.

According to an embodiment, the system may further comprise a carrier arrangement configured to conform to an outer surface of the neural tissue, wherein the first electrode arrangement and the second electrode arrangement are arranged in the carrier arrangement.

The carrier arrangement may be used for providing a well-defined relationship of the first electrode arrangement and the second electrode arrangement to each other and to the neural tissue. The carrier may also facilitate arrangement of the first electrode arrangement and the second electrode arrangement in relation to the neural tissue, such that implantation may be facilitated.

It should be realized that the first electrode arrangement and the second electrode arrangement need not necessarily be arranged in a common carrier arrangement. Rather, the system may comprise separate carriers in which the first and the second electrode arrangements are arranged.

According to an embodiment, the carrier arrangement comprises a first carrier unit and a second carrier unit, each configured to conform to the outer surface of the neural tissue, wherein the first electrode arrangement is arranged in the first carrier unit and the second electrode arrangement is arranged in the second carrier unit.

It should be realized that, as the system may comprise a plurality of electrode arrangements, each electrode arrangement may be arranged in a respective carrier unit. In other words, in one example the system may comprise a first, a second and a third electrode arrangement.

According to an embodiment, the carrier arrangement may have a form of a cuff configured to be arranged around a nerve.

The cuff-formed carrier arrangement provides a convenient manner of arranging the electrodes in relation to a cross-section of the nerve. The cuff may be easily mounted around the nerve, whereby the cuff will position the electrodes of the first pair of electrodes and the second pair of electrodes in appropriate positions in relation to the nerve. This facilitates implantation of the device.

According to a second aspect, there is provided a method for controlling selective nerve stimulation of neural tissue of a subject, said method comprising, providing a first intermittent current waveform comprising a first frequency to a first pair of electrodes of a first electrode arrangement at a first longitudinal position of the neural tissue, providing a second intermittent current waveform comprising a second frequency to a second pair of electrodes of the first electrode arrangement at the first longitudinal position of the neural tissue, stimulating a plurality of nerve fibers of the neural tissue using interferential stimulation based on interference between the first intermittent current waveform and the second intermittent current waveform, wherein the interferential stimulation of each of the nerve fibers of the plurality of nerve fibers is configured to activate stimulation of the nerve fiber at a specific time point, for initiating propagation of signals through the neural tissue towards a second longitudinal position, and providing a blocking pulse to a second electrode arrangement, for temporally selectively blocking of propagation of signals through the neural tissue at a second longitudinal position of the neural tissue.

This aspect may generally present the same or corresponding advantages as the former aspect. Effects and features of this second aspect are largely analogous to those described above in connection with the first aspect. Embodiments mentioned in relation to the second aspect are largely compatible with the first aspect.

Thanks to the method, accurate control of selective nerve stimulation is provided.

It should be realized that the interferential stimulation of each of the nerve fibers of the plurality of nerve fibers is configured to activate stimulation of the nerve fiber at a specific time point by providing an amplitude modulated interferential signal. Thus, a timing pattern of activation may be controlled. In other words, due to the properties of the fiber and the amplitude modulated signal a time delay is provided, and the timing pattern of activation may be controlled.

According to an embodiment, the method further comprises synchronizing the blocking pulse with respect to the interferential stimulation, such that temporally selectively blocking of propagation of signals through the neural tissue is achieved.

This implies that a timing of the blocking pulse may be controlled such that the blocking pulse may block propagation of undesired signals through the neural tissue.

It should be realized that synchronizing the blocking pulse with respect to the interferential stimulation may include providing a first intermittent current waveform comprising a first frequency to a first pair of electrodes of a first electrode arrangement and providing a second intermittent current waveform comprising a second frequency to a second pair of electrodes of the first electrode arrangement, and providing a blocking pulse to a second electrode arrangement after one another. Moreover, there may be an overlap, such that the blocking pulse may be provided substantially simultaneously as the first intermittent current waveform and the second intermittent current waveform is provided. In other words, the blocking pulse might start before the stimulation pulse is finished. This may be due to for example different electrode locations, conduction velocities of different fibers and membrane time-constants, and it may thus be beneficial to overlap the blocking pulse with the stimulation pulse, so as to provide improved stimulation control.

According to an embodiment, the method further comprising synchronizing the first pulse and the second pulse, such that an interferential signal is provided.

Thus, the first and second pulses are output such that the first and second pulses interfere in the neural tissue and form an interferential signal.

Thanks to synchronizing of the first and second pulses, interference provided in different portions of the neural tissue may be controlled, such that the interferential signal for stimulating the neural tissue may be steered to desired location in the neural tissue.

According to a third aspect, there is provided a device for controlling selective nerve stimulation of neural tissue of a subject, said device being configured to: control timing of generation of a first intermittent current waveform comprising a first frequency to be output to a first pair of electrodes of a first electrode arrangement at a first longitudinal position of the neural tissue, control timing of generation of a second intermittent current waveform comprising a second frequency to be output to a second pair of electrodes of the first electrode arrangement at the first longitudinal position of the neural tissue, wherein timing of the first and second intermittent current waveform controls timing of stimulation of a plurality of nerve fibers of the neural tissue using interferential stimulation based on interference between the first intermittent current waveform and the second intermittent current waveform, wherein the interferential stimulation of each of the nerve fibers of the plurality of nerve fibers is configured to activate stimulation of the nerve fiber at a specific time point, for initiating propagation of signals through the neural tissue towards a second longitudinal position; and control timing of generation of a blocking pulse to be output to a second electrode arrangement, for temporally selectively blocking of propagation of signals through the neural tissue at a second longitudinal position of the neural tissue.

This aspect may generally present the same or corresponding advantages as the former aspect. Effects and features of this third aspect are largely analogous to those described above in connection with the first and second aspects. Embodiments mentioned in relation to the third aspect are largely compatible with the first and second aspects.

The device may be implemented as a control unit which is configured to control timing of generation of signals. Thus, the control unit may ensure that a timing of a blocking pulse for selectively blocking propagation of signals is appropriately synchronized to the first and second intermittent current waveforms for causing interferential stimulation initiating propagation of signals through the neural tissue.

The control unit may thus be configured to provide a control for ensuring that undesired propagation of signals in certain nerve fibers may be blocked while desired propagation of signals in other nerve fibers may be allowed.

The controlling of timing of generation of signals may be achieved by the control unit sending a control signal to a stimulation signal generating unit for causing generation of a signal by the stimulation signal generating unit. Thus, the controlling of timing does not relate to actual output of the first and second intermittent current waveform or the blocking pulse, respectively, but rather relates to providing a timed control of the stimulation signal generating unit.

The control unit may be implemented in one or more general-purpose processing units, such as a central processing unit (CPU), which may execute instructions of one or more computer programs in order to implement functionality of the control unit. However, the control unit may alternatively be implemented as firmware arranged e.g., in an embedded system, or as a specifically designed processing unit, such as an Application-Specific Integrated Circuit (ASIC) or a Field-Programmable Gate Array (FPGA).

According to a fourth aspect, there is provided a method for controlling timing of selective nerve stimulation of neural tissue of a subject, said method comprising: controlling timing of generation of a first intermittent current waveform comprising a first frequency to be output to a first pair of electrodes of a first electrode arrangement at a first longitudinal position of the neural tissue, controlling timing of generation of a second intermittent current waveform comprising a second frequency to be output to a second pair of electrodes of the first electrode arrangement at the first longitudinal position of the neural tissue, wherein timing of the first and second intermittent current waveform controls timing of stimulation of a plurality of nerve fibers of the neural tissue using interferential stimulation based on interference between the first intermittent current waveform and the second intermittent current waveform, wherein the interferential stimulation of each of the nerve fibers of the plurality of nerve fibers is configured to activate stimulation of the nerve fiber at a specific time point, for initiating propagation of signals through the neural tissue towards a second longitudinal position; and controlling timing of generation of a blocking pulse to be output to a second electrode arrangement, for temporally selectively blocking of propagation of signals through the neural tissue at a second longitudinal position of the neural tissue.

This aspect may generally present the same or corresponding advantages as the former aspect. Effects and features of this fourth aspect are largely analogous to those described above in connection with the first, second, and third aspects. Embodiments mentioned in relation to the fourth aspect are largely compatible with the first, second, and third aspects.

The method may be performed by a control unit which is configured to control timing of generation of signals. The controlling of timing of generation of signals may be achieved by the control unit sending a control signal to a stimulation signal generating unit for causing generation of a signal by the stimulation signal generating unit. Thus, the controlling of timing does not relate to actual output of the first and second intermittent current waveform or the blocking pulse, respectively, but rather relates to providing a timed control of the stimulation signal generating unit.

According to a fifth aspect, there is provided a computer program product comprising computer-readable instructions which when executed by a processing unit cause the processing unit to perform the method according to the fourth aspect.

This aspect may generally present the same or corresponding advantages as the former aspect. Effects and features of this fifth aspect are largely analogous to those described above in connection with the first, second, third, and fourth aspects. Embodiments mentioned in relation to the fifth aspect are largely compatible with the first, second, third, and fourth aspects.

The computer program product may thus provide computer-readable instructions for allowing the method to be implemented. This allows the functionality of the method to be provided to any processing unit.

The computer program product may comprise a non-transient computer-readable medium for carrying the computer-readable instructions. Alternatively, in other embodiments, the computer program product may comprise a signal carrying the computer-readable instructions, e.g., for communicating the computer program product to the processing unit through wired or wireless communication.

### Brief description of the drawings

The above, as well as additional objects, features, and advantages of the present description, will be better understood through the following illustrative and non-limiting detailed description, with reference to the appended drawings. In the drawings like reference numerals will be used for like elements unless stated otherwise.
Fig. 1 is a schematic view illustrating a system for controlling selective stimulation of neural tissue of a subject.
Fig. 2a is a schematic view illustrating a detailed overview of a system for controlling selective stimulation of neural tissue of a subject.
Fig. 2b is a schematic view illustrating a first pulse and a second pulse.
Fig. 2c is a schematic view illustrating a portion of the interferential signal.
Fig. 2d is a schematic view illustrating signal propagation.
Fig. 3a, 3b and 3c are schematic views illustrating alternative embodiments of the system for controlling selective stimulation of neural tissue of a subject.
Fig. 4 schematically illustrates a method for controlling selective stimulation of neural tissue of a subject.
Fig. 5 schematically illustrates another method for controlling selective stimulation of neural tissue of a subject.

### Detailed description

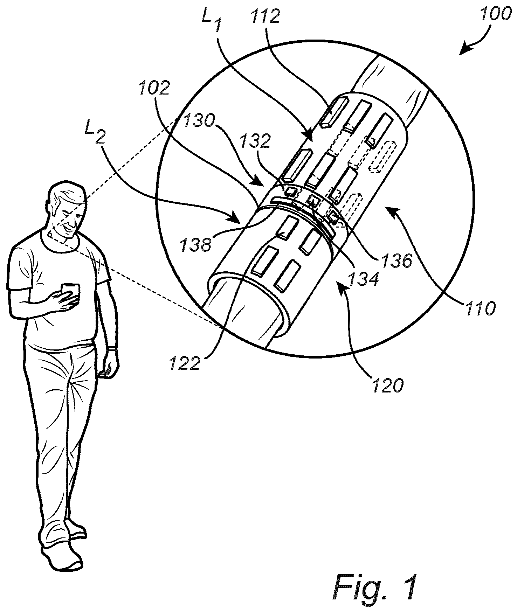

Referring now to Fig. 1, a system 100 for controlling selective stimulation of neural tissue of a subject will be described.

As illustrated in Fig. 1, the system 100 comprises a first electrode arrangement 110, a second electrode arrangement 120 and a stimulation signal generating unit 130.

The first electrode arrangement 110 may comprise a plurality of electrodes 112. The electrodes 112 of the plurality of electrodes are preferably arranged in pairs along the nerve.

The second electrode arrangement 120 may comprise at least one electrode 122.

In Fig. 1, the second electrode arrangement 120 is illustrated having a plurality of electrodes 122, in this case four electrodes 122. The electrodes 122 of the plurality of electrodes are preferably arranged in pairs.

The stimulation signal generating unit 130 may be connected to the first electrode arrangement 110 and/or the second electrode arrangement 120. The stimulation signal generating unit 130 may for instance be configured to generate a first intermittent current waveform comprising a first pulse and a second intermittent current waveform comprising a second pulse, and may be further configured to output the first intermittent current waveform to a first pair of electrodes of the first electrode arrangement 110 and the second intermittent current waveform to a second pair of electrodes of the first electrode arrangement 110, and thereby causing interferential stimulation of a plurality of nerve fibers of the neural tissue.

The first electrode arrangement 110 is configured to be implanted in a subject. The subject may be a human or animal. The first electrode arrangement 110 may be implanted at the brain, spinal cord or around any nerve in the body of the subject. As illustrated in Fig. 1, the first electrode arrangement 110 may be arranged around a vagus nerve in the subject. The electrodes 112 of the first electrode arrangement 110 are arranged in different electrode locations in relation to a first longitudinal position L₁ of the neural tissue.

As illustrated in Fig. 1, the electrodes 112 of the first electrode arrangement 110 may comprise a plurality of pairs of electrodes 112, wherein each pair comprises two electrodes displaced longitudinally in relation to each other along a nerve at which the first electrode arrangement 110 is arranged. The electrode arrangement 110 may thus be arranged in a carrier 102 formed as a cuff, which is arranged around the nerve for placing the electrodes 112 in relation to the nerve.

The electrode arrangement 110 may comprise a plurality of pairs of electrodes 112 arranged in different circumferential positions around the nerve. In Fig. 1, six pairs of electrodes 112 are illustrated arranged in six different locations around the nerve.

It should be realized that in other embodiments, other numbers of electrodes may be used and that a larger or a smaller number of electrodes may be used. In a simple embodiment, the electrode arrangement 110 comprises two pairs of electrodes 112.

Each of the pairs of electrodes 112 form a stimulation location, wherein a stimulation location is established between the first and the second electrode of the pair of electrodes 112.

It should be realized that the plurality of electrodes 112 may comprise an array of electrodes wherein a pair of electrodes for receiving a signal from the stimulation signal generating unit 130 may be dynamically selected when the signal is to be output to the electrodes 112.

The second electrode arrangement 120 is configured to be implanted in a subject. The subject may be a human or animal. The second electrode arrangement 120 may be implanted at the brain, spinal cord or around any nerve in the body of the subject. As mentioned above, the second electrode arrangement 120 comprises at least one electrode 122. The second electrode arrangement 120 may comprise a plurality of electrodes 122. In such case, the plurality of electrodes 122 of the second electrode arrangement 120 may be arranged in different electrode locations in relation to a second longitudinal position L₂ of the neural tissue.

The stimulation signal generating unit 130 may further be configured to generate a blocking pulse and to output the blocking pulse to the at least one electrode 122 of the second electrode arrangement 120. The blocking pulse may be output to a pair of electrodes 122 of the second electrode arrangement 120. Alternatively, for instance in case the second electrode arrangement 120 comprises only one electrode 122, the blocking pulse may be output to the electrode 122 of the second electrode arrangement 120 in relation to a reference electrode, which may be formed by one of the electrodes of the first electrode arrangement 110 or which may be a separate electrode. The blocking pulse may be provided to temporally selectively block propagation of signals through the neural tissue at the second longitudinal position L₂.

The stimulation signal generating unit 130 may be configured to generate the first intermittent current waveform, the second intermittent current waveform, and the blocking pulse. The stimulation signal generating unit 130 may comprise separate current generators for generating separate signals. This may imply that the generation of one waveform is not affected by generation of other waveforms.

For instance, the stimulation signal generating unit 130 may comprise a first current generator 132 configured to generate the first intermittent current waveform, a second current generator 134 configured to generate the second intermittent current waveform, and a third current generator 136 configured to generate the blocking pulse.

The system 100 may further comprise a control unit 138. The control unit 138 may be configured to control timing of generation and output of the first intermittent current waveform, the second intermittent current waveform and the blocking pulse by the stimulation signal generating unit 130. The control unit 138 may be implemented as an internal module within the signal generating unit 130 for providing a desired timing control of the generation and output of signals. The control unit 138 may alternatively be implemented as a separate unit.

The control unit 138 may for instance be implemented in a general-purpose processing unit, such as a central processing unit (CPU), which may execute instructions of one or more computer programs in order to implement functionality of the control unit 138. However, the control unit 138 may alternatively be implemented as firmware arranged e.g., in an embedded system, or as a specifically designed processing unit, such as an Application-Specific Integrated Circuit (ASIC) or a Field-Programmable Gate Array (FPGA).

The interferential stimulation of each of the nerve fibers of the plurality of nerve fibers may be configured to activate stimulation of the nerve fiber at a specific time point, and thereby initiating propagation of signals from the first longitudinal position, through the neural tissue towards the second longitudinal position.

The temporally selectively blocking of propagation of signals through the neural tissue is achieved at least by two components of the system. Firstly, the interferential stimulation by the first electrode arrangement activates neural tissues at different time points of the waveform of the interferential stimulation signal, depending on the location of the fiber with respect to the electric field. This causes different times of activation of different parts of the neural tissue, and thus a spatial differentiation. Secondly, the second electrode arrangement blocks the propagation of neural signals that are traveling through a second longitudinal position at the blocking electrode in the second arrangement, resulting in selective spatio-temporal deactivation of specific fibers in the neural tissue.

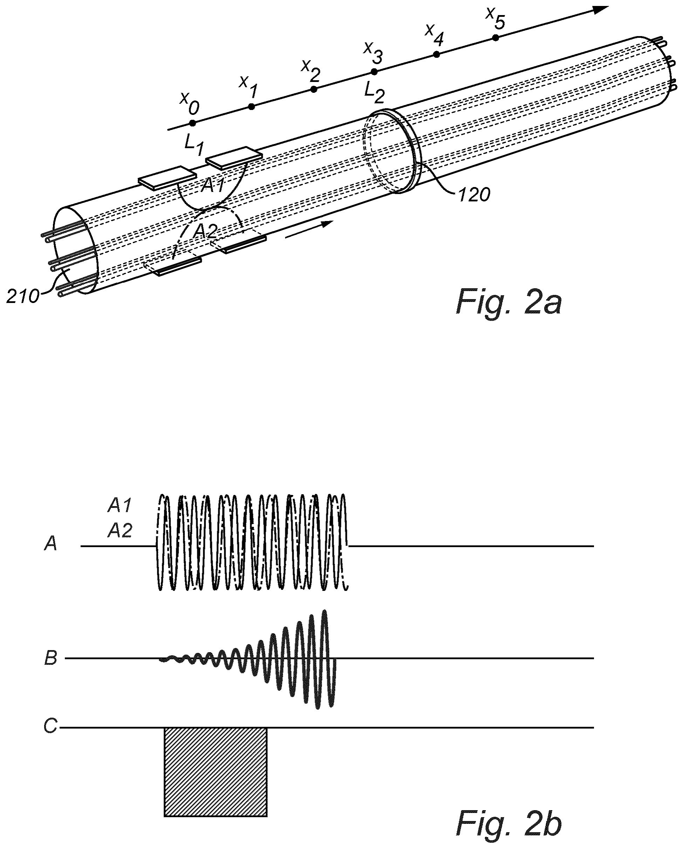

Referring now to Fig. 2a, a detailed overview of the system 100 for controlling of signals for selective stimulation of neural tissue of a subject will be described.

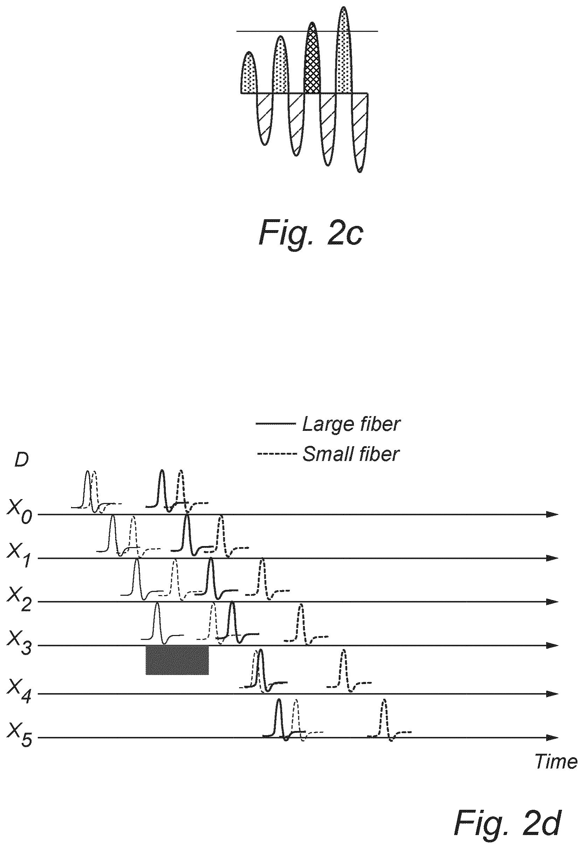

The first electrode arrangement 110 is illustrated arranged at a first longitudinal position L₁. In Fig. 2, two pairs of electrodes 112 are illustrated arranged in four different locations around the nerve 210, wherein each of the electrodes in a pair of electrodes are longitudinally displaced.

At a second longitudinal position L₂ the second electrode arrangement 120 is arranged. In Fig. 2, one electrode is illustrated comprised in the second electrode arrangement 120 extending along an entire circumference of the nerve.

The first longitudinal position L₁ and second longitudinal position L₂ are displaced along the nerve 210.

In a practical example, a first intermittent current waveform comprising a first pulse will be provided to the first pair of electrodes, i.e., outputted from the stimulation signal generating unit 130, and a second intermittent current waveform comprising a second pulse will be provided to the second pair of electrodes, i.e., outputted from the stimulation signal generating unit.

The first pulse comprises a first frequency and the second pulse comprises a second frequency, and the first frequency is different from the second frequency. The first and second pulse will interfere with each other, creating an interference waveform, that will cause interferential stimulation of a plurality of nerve fibers of the neural tissue.

As illustrated at A in Fig. 2b, a first pulse A1 and a second pulse A2 may be generated. The interference of the first pulse A1 with the second pulse A2 causes an interferential signal which has a beat frequency based on the difference in frequency between the first frequency and the second frequency.

The interferential signal is illustrated at B in Fig. 2b. The interferential signal is an amplitude modulated signal, wherein a sequence of cycles of varying amplitude are provided. A peak amplitude of the cycles varies such that an envelope curve is defined, which has a beat frequency corresponding to the difference between the first frequency and the second frequency.

As illustrated in Fig. 2b, the stimulation signal generating unit 130 may provide a time duration of the first pulse and the second pulse corresponding to half a period of the beat frequency. This may be a power efficient manner of providing the interferential signal for causing stimulation of nerve fibers in the nerve.

The stimulation signal generating unit 130 may be configured to generate the first intermittent current waveform with the first pulse comprising the first frequency and to generate the second intermittent current waveform with the second pulse comprising the second frequency. Each of the first pulse and the second pulse may be a sinusoidal signal having the first frequency and the second frequency, respectively.

The stimulation signal generating unit 130 may further be configured to generate the first intermittent current waveform and to generate the second intermittent current waveform with a difference in frequency between the signals in the first pulse and the second pulse. The difference between the first frequency of the signal in the first pulse and the second frequency of the signal in the second pulse may be in a range of 1 Hz - 10 kHz, such as 1 - 5 kHz. The difference in frequency defines the beat frequency of the interferential signal.

The beat frequency may be relatively low compared to the first frequency and the second frequency, respectively.

A portion of the interferential signal is illustrated in Fig. 2c. The peak amplitude of cycles is compared to an activation threshold. As evident from Fig. 2c, the peak amplitude is below the activation threshold for some cycles before reaching the activation threshold in a cycle (illustrated in shading in Fig. 2c). Thus, the interferential signal may activate stimulation of a nerve fiber when a cycle of the interferential signal reaches the activation threshold, such that an action potential is triggered in the nerve fiber. Subsequent cycles will not trigger action potentials due to a refractory period of the nerve fiber, if the subsequent cycles are within said refractory period.

The interferential signal may provide a time delay of fiber activation, which may be different for different nerve fibers. The time delay of activating different nerve fibers may be enhanced by interferential intermittent current stimulation due to the amplitude modulation of the interferential signal, which can result in fiber activation at different cycles within the envelope curve for different nerve fibers.

The interferential stimulation will cause activation of the plurality of nerve fibers within the neural tissue or part of the neural tissue. The activation of the nerve fibers will cause propagation of neural signal through the nerve fibers.

Due to the interferential signal/waveform having an increasing amplitude (i.e., being amplitude modulated) different nerve fibers will be activated at different time points. In addition, the plurality of nerve fibers may have different thresholds for activation further causing different nerve fibers to be activated at different time points. The activation threshold may for example depend on the degree of myelination or the circumference of the nerve fiber.

This will cause a time delay for the propagating signal between each of the plurality of nerve fibers. This may be utilized in providing the blocking pulse by the electrodes 122 of the second electrode arrangement 120 for temporally selectively blocking propagation of signals. Thus, propagation of undesired signals, such as signals propagating in off-target fibers may be blocked.

To further illustrate the time delay of the nerve fiber activation and signal propagation it is now referred to Fig. 2d, providing a schematical illustration of signal propagation.

In Fig. 2d, propagation of signals through longitudinal positions of the nerve 210 from position x₀ to position x₅, as indicated in Fig. 2a, is illustrated. For each longitudinal position, a time line is shown illustrating signals passing the longitudinal position as a function of time. Throughout Fig. 2d the thicker lines are intended to represent fibers located centrally within the nerve, while the thin lines are intended to represent more superficial fibers, and thus a time delay as illustrated may occur.

A first longitudinal position, L1, corresponds to a first position x₀ on the nerve. As illustrated in the time line of position x₀, non-center nerve fibers arranged close to the electrodes, i.e., close to a surface of the nerve, are activated first. In addition, large fibers are activated slightly before small fibers in the non-center location in the nerve 210. As further illustrated in the time line of position x₀, central nerve fibers arranged deep inside the nerve are activated at a later time point. Again, large fibers are activated slightly before small fibers in the central location in the nerve 210.

Thus, the interferential stimulation is configured to activate stimulation of nerve fibers at specific time points for initiating propagation of signals through the neural tissue towards the second longitudinal position L₂.

Fig. 2d further illustrates propagation of the signals in the nerve fibers. As illustrated in the time lines for positions x₁ and x₂ in the nerve, the speed of propagation of signals differ between different nerve fibers. Thus, a difference in time points at which signals of different nerve fibers pass the positions x₁ and x₂ may be larger than the difference in activation time points in position x₀.

As discussed above, the stimulation signal generating unit 130 is configured to generate a blocking pulse which is illustrated at C in Fig. 2b. The blocking pulse may be output to the at least one electrode 122 of the second electrode arrangement 120 for providing a blocking pulse at the second longitudinal position L₂, corresponding to position x₃ on the nerve.

The blocking pulse may be configured to block propagation of signals at the second longitudinal position L₂. Thus, signals passing the second longitudinal position L₂ during the time of output of the blocking pulse may be prevented from continuing propagation along the nerve fiber. A timing of the blocking pulse may thus be controlled to ensure temporally selective blocking of propagation of signals for blocking undesired signals that were triggered by the interferential stimulation in the first longitudinal position L₁.

As shown in the time line corresponding to position x₃ in Fig. 2d, the blocking pulse may be output during a time period when the signal propagating in large, non-center fibers arrive at the second longitudinal position. Thus, signals arriving early at the second longitudinal position are blocked. The blocking pulse may have a pulse width defining a time duration during which temporally selective blocking of propagation of signals through the nerve occurs. After this time duration, signals are not affected at the second longitudinal position allowing signals to propagate further.

This is illustrated in the time lines corresponding to positions x₄ and x₅ in Fig. 2d, where there is no longer any signal propagating in large, non-center fibers, but the other signals are propagating to these positions.

According to an embodiment, the blocking pulse may be configured to hyperpolarize the neural tissue at the second longitudinal position, such that the propagation of signals through the neural tissue may be prevented.

Hyperpolarization may be induced by applying a voltage, and thus changing a cell's membrane potential by decreasing the membrane potential. By sufficiently decreasing the membrane potential, action potentials transmitted will not be further propagated as regeneration of the action potential fails, due to the decreased membrane potential.

The blocking pulse may thus be configured to output a negative voltage at the second longitudinal position, as illustrated at C in Fig. 2b. The blocking pulse is illustrated in Fig. 2b as having a square-shaped waveform. However, it should be realized that the blocking pulse may have different waveforms, such as linear, triangular or trapezoidal. The pulse width of the blocking pulse may be adapted to the desired time duration during which blocking of propagation of signals is desired in the second longitudinal position.

It should be realized that a timing of start of the blocking pulse in relation to a timing of the interferential stimulation may vary. The start of the blocking pulse may be before or after start of the first and second intermittent current waveforms for causing the interferential stimulation.

According to an embodiment, the blocking pulse is achieved by anodal blocking.

In one example, anodal blocking may be achieved by utilizing one electrode of a pair of electrodes of the first electrode arrangement together with the electrode of the second electrode arrangement. The blocking pulse may be output to an electrode in the second electrode arrangement acting as an anode and to an electrode in the first electrode arrangement acting as a cathode, for forming the blocking pulse at the second electrode arrangement. In other words, an electrode of the first electrode arrangement may act both as receiver of the first or the second intermittent current waveform and as receiver of the blocking pulse.

According to an embodiment, the stimulation signal generating unit is configured to generate a repolarization pulse and to output the repolarization pulse to the at least one electrode of the second electrode arrangement following the blocking pulse.

The repolarization pulse may be beneficial to ensure charge balancing at the at least one electrode 122 of the second electrode arrangement 120.

An unbalanced charge may introduce an offset voltage build-up onto the electrode over time, in particular as the interface between the electrode and tissue is often very capacitive. The unbalanced charge can cause safety issues to the electrode and/or the tissue. For instance, the offset voltage build-up may form such a large electric field that electrical breakdown of water occurs. The offset voltage build-up may also cause the electrode to break such that a subject to the stimulation may be exposed to toxic substances.

The repolarization pulse may ensure that a charge balancing is provided by the hyperpolarization pulse and the repolarization pulse. Thus, an amplitude and time duration of the repolarization pulse may be adapted to provide charge balancing of the build-up of charges by the amplitude and time duration of the hyperpolarization pulse.

The blocking pulse may thus have a biphasic waveform, wherein the repolarization pulse may be applied immediately following the hyperpolarization pulse or with some delay therebetween.

The repolarization pulse may have a smaller amplitude than the hyperpolarization pulse in order not to affect signal propagation in the nerve. Hence, the repolarization pulse may have a correspondingly longer pulse width. It should be realized that the repolarization pulse may have a square-shaped waveform but may alternatively have another waveform, such as linear, triangular or trapezoidal.

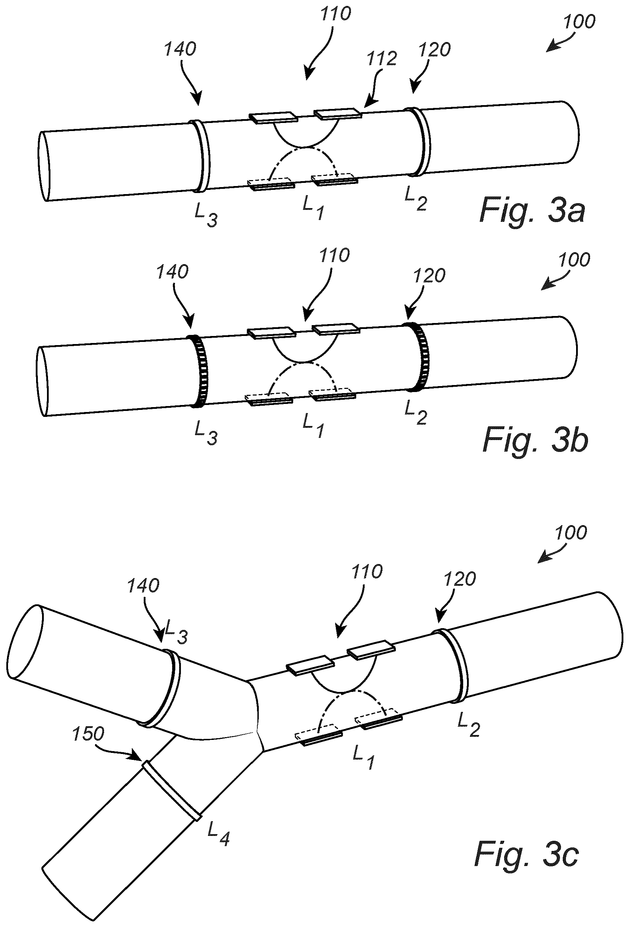

Fig. 3a, 3b, 3c and 3d are schematic views illustrating alternative embodiments of the system 100.

Referring now to Fig. 3a, a first alternative view of the system 100 is shown. The first electrode arrangement 110 is illustrated arranged at a first longitudinal position L₁. In Fig. 3a, two pairs of electrodes 112 are illustrated arranged in four different locations around the nerve, wherein each of the electrodes in a pair of electrodes are longitudinally displaced.

At a second longitudinal position L₂ the second electrode arrangement 120 is arranged. In Fig. 3a, one electrode is illustrated comprised in the second electrode arrangement 120.

At a third longitudinal position L₃ a third electrode arrangement 140 is arranged. In Fig. 3a, one electrode is illustrated comprised in the third electrode arrangement 140.

The second and third electrode arrangements 120, 140 are illustrated comprising one electrode each, wherein the electrode is illustrated encircling the nerve 210.

The second and third electrode arrangements 120, 140 are arranged on opposite sides of the first electrode arrangement 110. This means the second electrode arrangement is configured to block signal propagation in a first direction from the first longitudinal position L₁, and the third electrode arrangement is configured to block signal propagation in a second direction from the first longitudinal position L₁. The first direction is opposite to the second direction.

Referring now to Fig. 3b, a second alternative view of the system 100 is shown. The first electrode arrangement 110 is illustrated arranged at a first longitudinal position L₁. In Fig. 3b, two pairs of electrodes 112 are illustrated arranged in four different locations around the nerve, wherein each of the electrodes in a pair of electrodes are longitudinally displaced.

At a second longitudinal position L₂ the second electrode arrangement 120 is arranged. In Fig. 3b, a plurality of electrodes are illustrated comprised in the second electrode arrangement 120.

At a third longitudinal position L₃ a third electrode arrangement 140 is arranged. In Fig. 3b, a plurality of electrodes are illustrated comprised in the third electrode arrangement 140.

The second and third electrode arrangements 120, 140 are arranged on opposite sides of the first electrode arrangement 110. This means the second electrode arrangement 120 is configured to block signal propagation in a first direction from the first longitudinal position L₁, and the third electrode arrangement 140 is configured to block signal propagation in a second direction from the first longitudinal position L₁. The first direction is opposite to the second direction.

The second and third electrode arrangements 120, 140 are illustrated comprising plurality of electrodes each. The plurality of electrodes are arranged at different electrode location adjacent the nerve 210, such that they at least partially encircle the nerve. Each of this plurality of electrodes at electrode arrangements 120 and 140 can be individually used to deliver the blocking pulse, such that any subset of those electrodes can be used to confine the blocking predominantly in the area of the nerve that is close to this subset of electrodes.

Referring now to Fig. 3c, a third alternative view of the system 100 is shown. The first electrode arrangement 110 is illustrated arranged at a first longitudinal position L₁. In Fig. 3c, two pairs of electrodes 112 are illustrated arranged in four different locations around the nerve, wherein each of the electrodes in a pair of electrodes are longitudinally displaced.

At a second longitudinal position L₂ the second electrode arrangement 120 is arranged. In Fig. 3c, one electrode is illustrated comprised in the second electrode arrangement 120.

At a third longitudinal position L₃ a third electrode arrangement 140 is arranged. In Fig. 3c, one electrode is illustrated comprised in the third electrode arrangement 140.

At a fourth longitudinal position L₄ a fourth electrode arrangement 150 is arranged. In Fig. 3c, one electrode is illustrated comprised in the fourth electrode arrangement 150.

The second electrode arrangement is arranged at a first side of the first electrode arrangement, such that the second electrode arrangement 120 is configured to block signal propagation in a first direction from the first longitudinal position L₁. The third and fourth electrode arrangements 140, 150 are arranged on the opposite side of the first electrode arrangement 110, respective to the second electrode arrangement 120. The third and fourth electrode arrangements 140, 150 are configured to block signal propagation in a second direction from the first longitudinal position L₁. The first direction is opposite the second direction.

Fig. 3c further illustrates the nerve 210 branching, and the third electrode arrangement 140 is arranged at a first branch and the fourth electrode arrangement 150 is arranged at a second branch for providing selective blocking of signal propagation in the first branch and in the second branch, respectively.

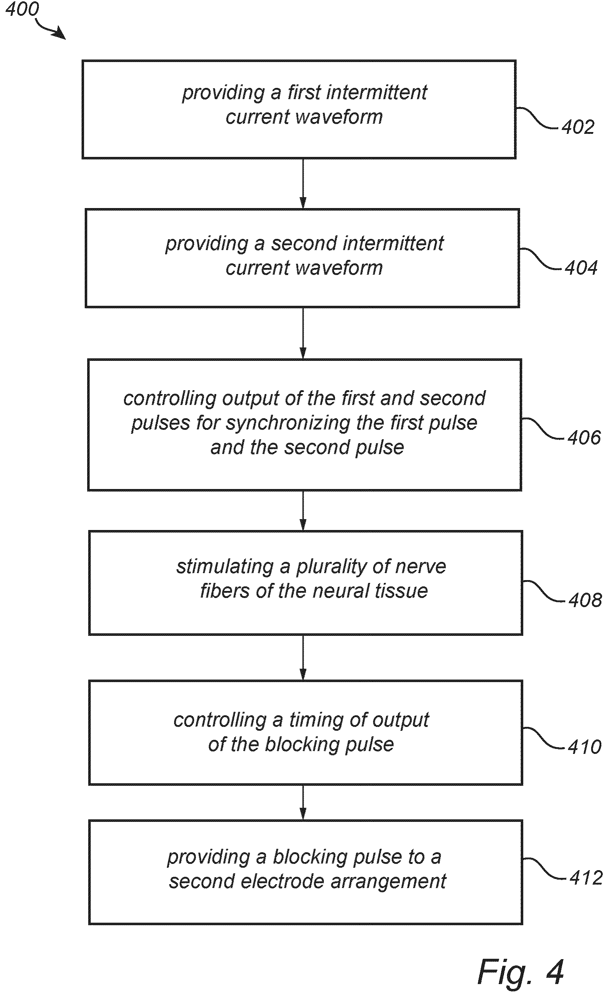

Referring now to Fig. 4, a schematical illustration of a method 400 for controlling selective nerve stimulation of neural tissue of a subject is shown.

As indicated by block 402, the method 400 comprises providing a first intermittent current waveform comprising a first frequency to a first pair of electrodes of a first electrode arrangement at a first longitudinal position of the neural tissue, and, as indicated by block 404, the method 400 further comprises providing a second intermittent current waveform comprising a second frequency to a second pair of electrodes of the first electrode arrangement at the first longitudinal position of the neural tissue.

It should be realized that providing 402 a first intermittent current waveform and providing 404 a second intermittent current waveform may occur substantially simultaneously.

As indicated by block 406, the method 400 may also comprise controlling output of the first and second pulses for synchronizing the first pulse and the second pulse, such as to control an interferential signal in neural tissue based on the first and second pulses.

Thereafter, as indicated by block 408, the method further comprises, stimulating a plurality of nerve fibers of the neural tissue using interferential stimulation based on interference between the first intermittent current waveform and the second intermittent current waveform. The interferential stimulation of each of the nerve fibers of the plurality of nerve fibers is configured to activate stimulation of the nerve fiber at a specific time point, for initiating propagation of signals through the neural tissue towards a second longitudinal position.

Lastly, the method 400 comprises providing a blocking pulse to a second electrode arrangement, for temporally selectively blocking of propagation of signals through the neural tissue at a second longitudinal position of the neural tissue, as indicated by block 412.

As indicated by block 410, the method 400 may further comprise, before providing the blocking pulse, controlling a timing of output of the blocking pulse for synchronizing the blocking pulse with respect to the interferential stimulation, such that a desired temporally selective blocking of propagation of signals through the neural tissue is achieved.

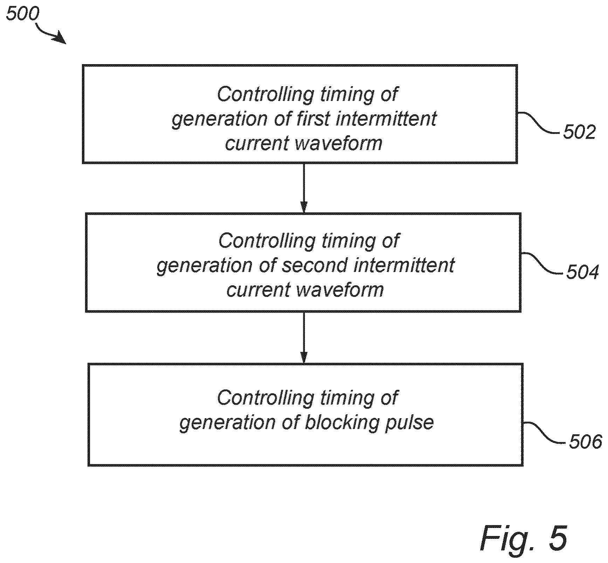

Referring now to Fig. 5, a schematical illustration of a method 500 for controlling selective nerve stimulation of neural tissue of a subject is shown.

The method may be implemented by software and/or hardware for controlling generation and output of signals for providing selective nerve stimulation of neural tissue of the subject.

The method comprises controlling 502 timing of generation of a first intermittent current waveform comprising a first frequency to be output to a first pair of electrodes of a first electrode arrangement at a first longitudinal position of the neural tissue.

The method further comprises controlling 504 timing of generation of a second intermittent current waveform comprising a second frequency to be output to a second pair of electrodes of the first electrode arrangement at the first longitudinal position of the neural tissue.

The first and second intermittent current waveforms cause interferential stimulation. Thus, timing of the first and second intermittent current waveform controls timing of stimulation of a plurality of nerve fibers of the neural tissue using interferential stimulation based on interference between the first intermittent current waveform and the second intermittent current waveform. The interferential stimulation of each of the nerve fibers of the plurality of nerve fibers is configured to activate stimulation of the nerve fiber at a specific time point, for initiating propagation of signals through the neural tissue towards a second longitudinal position.

The method further comprises controlling 506 timing of generation of a blocking pulse to be output to a second electrode arrangement, for temporally selectively blocking of propagation of signals through the neural tissue at a second longitudinal position of the neural tissue.

Thus, the timing of the blocking pulse in relation to the interferential stimulation may be set to ensure that undesired propagation of signals in certain nerve fibers may be blocked while desired propagation of signals in other nerve fibers may be allowed.

The timing of generation of signals may be controlled by output of control signals by the control unit 138.

### Itemized list of embodiments

1. A system for controlling selective stimulation of neural tissue of a subject, said system comprising:
   a first electrode arrangement, comprising a plurality of electrodes, wherein different electrodes of the plurality of electrodes are configured to be arranged in different electrode locations in relation to a first longitudinal position of the neural tissue;
   a second electrode arrangement, comprising at least one electrode and configured to be arranged at a second longitudinal position of the neural tissue; and
   a stimulation signal generating unit,
   wherein the stimulation signal generating unit is configured to generate a first intermittent current waveform comprising a first pulse and a second intermittent current waveform comprising a second pulse, wherein the first pulse comprises a first frequency and the second pulse comprises a second frequency, wherein the second frequency is different from the first frequency,
   wherein the stimulation signal generating unit is configured to output the first intermittent current waveform to a first pair of electrodes of the first electrode arrangement and the second intermittent current waveform to a second pair of electrodes of the first electrode arrangement, for causing interferential stimulation of a plurality of nerve fibers of the neural tissue, wherein the interferential stimulation of each of the nerve fibers of the plurality of nerve fibers is configured to activate stimulation of the nerve fiber at a specific time point, for initiating propagation of signals through the neural tissue towards the second longitudinal position,
   wherein the stimulation signal generating unit is configured to generate a blocking pulse and to output the blocking pulse to the at least one electrode of the second electrode arrangement, for temporally selectively blocking propagation of signals through the neural tissue at the second longitudinal position.
2. The system according to embodiment 1, wherein the blocking pulse is configured to hyperpolarize the neural tissue at the second longitudinal position, such that the propagation of signals through the neural tissue is prevented.
3. The system according to any one of the preceding embodiments, wherein the stimulation signal generating unit is configured to generate the blocking pulse comprising at least a pulse width for defining a time duration during which temporally selectively blocking propagation of signals through the neural tissue occurs.
4. The system according to any one of the preceding embodiments, wherein the blocking pulse is achieved by anodal blocking.
5. The system according to any one of the preceding embodiments, wherein the second electrode arrangement comprises a plurality of electrodes, and wherein different electrodes of the plurality of electrodes are configured to be arranged in different electrode locations in relation to the second longitudinal position of the neural tissue.
6. The system according to any one of the preceding embodiments, wherein the stimulation signal generating unit is configured to generate a cathodic repolarization pulse and to output the cathodic repolarization pulse to the at least one electrode of the second electrode arrangement following the blocking pulse.
7. The system according to any one of the preceding embodiments, wherein the system further comprises a third electrode arrangement, comprising at least one electrode and configured to be arranged at a third longitudinal position of the neural tissue, and wherein the second longitudinal position of the neural tissue is located in a first direction along the neural tissue in relation to the first longitudinal position and the third longitudinal position of the neural tissue is located in a second direction along the neural tissue in relation to the first longitudinal position, wherein the second direction is opposite to the first direction.
8. The system according to any one of the preceding embodiments, wherein the stimulation signal generating unit comprises
   a first current generator, configured to generate the first intermittent current waveform and
   a second current generator, configured to generate the second intermittent current waveform.
9. A system according to embodiment 8, wherein the stimulation signal generating unit comprises a third current generator, configured to generate the blocking pulse.
10. The system according to any one of the preceding embodiments, wherein the system is configured to be at least partially implanted in the subject.
11. The system according to any one of the preceding embodiments, wherein the stimulation generating unit is configured to generate a first frequency and a second frequency, respectively, being in a range of 100 Hz-1 MHz, such as 10 kHz-100 kHz.
12.The system according to any one of the preceding embodiments, wherein the stimulation signal generating unit is configured to generate the first intermittent current waveform and the second intermittent current waveform with a difference in frequency in a range of 1 Hz - 10 kHz, such as 1 - 5 kHz.
13. The system according to any one of the preceding embodiments, wherein the stimulation signal generating unit is configured to generate the first intermittent current waveform and the second intermittent current waveform with a time duration of the first pulse and the second pulse corresponding to a portion of a period of a beat frequency.
14.The system according to any one of the preceding embodiments, wherein the first pulse and the second pulse are sinusoidal signals.
15. The system according to any one of the preceding embodiments, further comprising a carrier arrangement configured to conform to an outer surface of the neural tissue, wherein the first electrode arrangement and the second electrode arrangement are arranged in the carrier arrangement.
16. The system according to embodiment 15, wherein the carrier arrangement comprises a first carrier unit and a second carrier unit, each configured to conform to the outer surface of the neural tissue, wherein the first electrode arrangement is arranged in the first carrier unit and the second electrode arrangement is arranged in the second carrier unit.
17. The system according to embodiment 15, wherein the carrier arrangement has a form of a cuff configured to be arranged around a nerve.
18. A method for controlling selective nerve stimulation of neural tissue of a subject, said method comprising:
   providing a first intermittent current waveform comprising a first frequency to a first pair of electrodes of a first electrode arrangement at a first longitudinal position of the neural tissue,
   providing a second intermittent current waveform comprising a second frequency to a second pair of electrodes of the first electrode arrangement at the first longitudinal position of the neural tissue,
   stimulating a plurality of nerve fibers of the neural tissue using interferential stimulation based on interference between the first intermittent current waveform and the second intermittent current waveform, wherein the interferential stimulation of each of the nerve fibers of the plurality of nerve fibers is configured to activate stimulation of the nerve fiber at a specific time point, for initiating propagation of signals through the neural tissue towards a second longitudinal position; and
   providing a blocking pulse to a second electrode arrangement, for temporally selectively blocking of propagation of signals through the neural tissue at a second longitudinal position of the neural tissue.
19. The method according to embodiment 18, further comprising synchronizing the blocking pulse with respect to the interferential stimulation, such that temporally selectively blocking of propagation of signals through the neural tissue is achieved.
20. The method according to embodiment 18 or embodiment 19, further comprising synchronizing the first pulse and the second pulse, such that an interferential signal is provided.
21. A device for controlling selective nerve stimulation of neural tissue of a subject, said device being configured to:
   control timing of generation of a first intermittent current waveform comprising a first frequency to be output to a first pair of electrodes of a first electrode arrangement at a first longitudinal position of the neural tissue,
   control timing of generation of a second intermittent current waveform comprising a second frequency to be output to a second pair of electrodes of the first electrode arrangement at the first longitudinal position of the neural tissue, wherein timing of the first and second intermittent current waveform controls timing of stimulation of a plurality of nerve fibers of the neural tissue using interferential stimulation based on interference between the first intermittent current waveform and the second intermittent current waveform, wherein the interferential stimulation of each of the nerve fibers of the plurality of nerve fibers is configured to activate stimulation of the nerve fiber at a specific time point, for initiating propagation of signals through the neural tissue towards a second longitudinal position; and
   control timing of generation of a blocking pulse to be output to a second electrode arrangement, for temporally selectively blocking of propagation of signals through the neural tissue at a second longitudinal position of the neural tissue.
22. A method for controlling selective nerve stimulation of neural tissue of a subject, said method comprising:
   controlling timing of generation of a first intermittent current waveform comprising a first frequency to be output to a first pair of electrodes of a first electrode arrangement at a first longitudinal position of the neural tissue,
   controlling timing of generation of a second intermittent current waveform comprising a second frequency to be output to a second pair of electrodes of the first electrode arrangement at the first longitudinal position of the neural tissue, wherein timing of the first and second intermittent current waveform controls timing of stimulation of a plurality of nerve fibers of the neural tissue using interferential stimulation based on interference between the first intermittent current waveform and the second intermittent current waveform, wherein the interferential stimulation of each of the nerve fibers of the plurality of nerve fibers is configured to activate stimulation of the nerve fiber at a specific time point, for initiating propagation of signals through the neural tissue towards a second longitudinal position; and
   controlling timing of generation of a blocking pulse to be output to a second electrode arrangement, for temporally selectively blocking of propagation of signals through the neural tissue at a second longitudinal position of the neural tissue.
23. A computer program product comprising computer-readable instructions which when executed by a processing unit cause the processing unit to perform the method according to embodiment 22.

In the above the inventive concept has mainly been described with reference to a limited number of examples. However, as is readily appreciated by a person skilled in the art, other examples than the ones disclosed above are equally possible within the scope of the inventive concept, as defined by the appended claims.

## Claims

1. A system for controlling selective stimulation of neural tissue of a subject, said system comprising:
a first electrode arrangement, comprising a plurality of electrodes, wherein different electrodes of the plurality of electrodes are configured to be arranged in different electrode locations in relation to a first longitudinal position of the neural tissue;
a second electrode arrangement, comprising at least one electrode and configured to be arranged at a second longitudinal position of the neural tissue; and
a stimulation signal generating unit,
wherein the stimulation signal generating unit is configured to generate a first intermittent current waveform comprising a first pulse and a second intermittent current waveform comprising a second pulse, wherein the first pulse comprises a first frequency and the second pulse comprises a second frequency, wherein the second frequency is different from the first frequency,
wherein the stimulation signal generating unit is configured to output the first intermittent current waveform to a first pair of electrodes of the first electrode arrangement and the second intermittent current waveform to a second pair of electrodes of the first electrode arrangement, thereby causing interferential stimulation of a plurality of nerve fibers of the neural tissue, wherein the interferential stimulation of each of the nerve fibers of the plurality of nerve fibers is configured to activate stimulation of the nerve fiber at a specific time point, thereby initiating propagation of signals through the neural tissue towards the second longitudinal position,
wherein the stimulation signal generating unit is configured to generate a blocking pulse and to output the blocking pulse to the at least one electrode of the second electrode arrangement, thereby temporally selectively blocking propagation of signals through the neural tissue at the second longitudinal position.

2. The system according to claim 1, wherein the blocking pulse is configured to hyperpolarize the neural tissue at the second longitudinal position, such that the propagation of signals through the neural tissue is prevented.

3. The system according to claim 1 or 2, wherein the stimulation signal generating unit is configured to generate the blocking pulse comprising at least a pulse width for defining a time duration during which temporally selectively blocking propagation of signals through the neural tissue occurs.

4. The system according to any one of the preceding claims, wherein the blocking pulse is achieved by anodal blocking.

5. The system according to any one of the preceding claims, wherein the second electrode arrangement comprises a plurality of electrodes, and wherein different electrodes of the plurality of electrodes are configured to be arranged in different electrode locations in relation to the second longitudinal position of the neural tissue.

6. The system according to any one of the preceding claims, wherein the stimulation signal generating unit is configured to generate a repolarization pulse and to output the repolarization pulse to the at least one electrode of the second electrode arrangement following the blocking pulse.

7. The system according to any one of the preceding claims, wherein the system further comprises a third electrode arrangement, comprising at least one electrode and configured to be arranged at a third longitudinal position of the neural tissue, and wherein the second longitudinal position of the neural tissue is located in a first direction along the neural tissue in relation to the first longitudinal position and the third longitudinal position of the neural tissue is located in a second direction along the neural tissue in relation to the first longitudinal position, wherein the second direction is opposite to the first direction.

8. The system according to any one of the preceding claims, wherein the stimulation signal generating unit comprises
a first current generator, configured to generate the first intermittent current waveform;
a second current generator, configured to generate the second intermittent current waveform; and
a third current generator, configured to generate the blocking pulse.

9. The system according to any one of the preceding claims, wherein the system is configured to be at least partially implanted in the subject.

10. The system according to any one of the preceding claims, wherein the stimulation generating unit is configured to generate a first frequency and a second frequency, respectively, being in a range of 100 Hz-1 MHz, such as 10 kHz-100 kHz, and wherein the stimulation signal generating unit is configured to generate the first intermittent current waveform and the second intermittent current waveform with a difference in frequency in a range of 1 Hz - 10 kHz, such as 1 - 5 kHz.

11. The system according to any one of the preceding claims, wherein the stimulation signal generating unit is configured to generate the first intermittent current waveform and the second intermittent current waveform with a time duration of the first pulse and the second pulse corresponding to a portion of a period of a beat frequency.

12. The system according to any one of the preceding claims, wherein the first pulse and the second pulse are sinusoidal signals.

13. The system according to any one of the preceding claims, further comprising a carrier arrangement configured to conform to an outer surface of the neural tissue, wherein the first electrode arrangement and the second electrode arrangement are arranged in the carrier arrangement, wherein the carrier arrangement comprises a first carrier unit and a second carrier unit, each configured to conform to the outer surface of the neural tissue, wherein the first electrode arrangement is arranged in the first carrier unit and the second electrode arrangement is arranged in the second carrier unit.

14. A method for controlling timing of selective nerve stimulation of neural tissue of a subject, said method comprising:
controlling timing of generation of a first intermittent current waveform comprising a first frequency to be output to a first pair of electrodes of a first electrode arrangement at a first longitudinal position of the neural tissue,
controlling timing of generation of a second intermittent current waveform comprising a second frequency to be output to a second pair of electrodes of the first electrode arrangement at the first longitudinal position of the neural tissue, wherein timing of the first and second intermittent current waveform controls timing of stimulation of a plurality of nerve fibers of the neural tissue using interferential stimulation based on interference between the first intermittent current waveform and the second intermittent current waveform, wherein the interferential stimulation of each of the nerve fibers of the plurality of nerve fibers is configured to activate stimulation of the nerve fiber at a specific time point, for initiating propagation of signals through the neural tissue towards a second longitudinal position; and
controlling timing of generation of a blocking pulse to be output to a second electrode arrangement, for temporally selectively blocking of propagation of signals through the neural tissue at a second longitudinal position of the neural tissue.

15. A computer program product comprising computer-readable instructions which when executed by a processing unit cause the processing unit to perform the method according to claim 14.
